# EUROPEAN PATENT APPLICATION

(11) **EP 1 335 298 A2**
(43) Date of publication of application: **13.08.2003**
(21) Application number: 03250575.2
(22) Date of filing: 30.01.2003
(51) Int. Cl.: G06F 17/00, C07H 21/00, C12Q 1/68, B01J 19/00

(54) **Reading multiple chemical arrays**

(30) Priority: 30.01.2002 US 66516
(71) Applicant: Agilent Technologies, Inc., Palo Alto, CA 94306 (US)
(72) Inventor: Cattell, Herbert F., Mountain View, CA 94040-4545 (US)
(74) Representative: Tollett, Ian

(57) **Abstract**

A method, apparatus and computer program products relating to the reading of chemical arrays 12 and extracting feature characteristics therefrom. In a method multiple chemical arrays each having a plurality of features 16, are read to obtain array signal data. The array signal data for the multiple arrays is saved in a memory 300. The saved signal data for chemical arrays is retrieved from the memory and feature characteristics extracted therefrom, wherein the saved signal data for a chemical array is extracted while another chemical array is being read.

## Description

This invention relates to a method and apparatus for reading multiple arrays, particularly biopolymer arrays such as DNA arrays, which are useful in diagnostic, screening, gene expression analysis, and other applications.

Polynucleotide arrays (such as DNA, RNA, or protein arrays), are known and are useful, for example, as screening or diagnostic tools. Such arrays include regions of usually different sequence polynucleotides arranged in a predetermined configuration on a substrate. These regions (sometimes referenced as "features") are positioned at respective locations ("addresses") on the substrate. The arrays, when exposed to a sample, will exhibit an observed binding pattern. This binding pattern can be detected upon interrogating the array. For example all polynucleotide targets (for example, DNA) in the sample can be labeled with a suitable label (such as a fluorescent compound), and the fluorescence pattern on the array accurately observed following exposure to the sample. Assuming that the different sequence polynucleotides were correctly deposited in accordance with the predetermined configuration, then the observed binding pattern will be indicative of the presence and/or concentration of one or more polynucleotide components of the sample. Polynucleotide or other biopolymer arrays, can be fabricated by depositing previously obtained biopolymers (such as from synthesis or natural sources) onto a substrate, or by *in situ* synthesis methods. Methods of depositing obtained biopolymers include dispensing droplets to a substrate from dispensers such as pin or capillaries (such as described in US 5,807,522) or such as pulse jets (such as a piezoelectric inkjet head, as described in PCT publications WO 95/25116 and WO 98/41531, and elsewhere). The substrate is coated with a suitable linking layer prior to deposition, such as with polylysine or other suitable coatings as described, for example, in U.S. Patent 6,077,674 and the references cited therein.

For *in situ* fabrication methods, multiple different reagent droplets are deposited from drop dispensers at a given target location in order to form the final feature (hence a probe of the feature is synthesized on the array stubstrate). The *in situ* fabrication methods include those described in US 5,449,754 for synthesizing peptide arrays, and described in WO 98/41531 and the references cited therein for polynucleotides. The *in situ* method for fabricating a polynucleotide array typically follows, at each of the multiple different addresses at which features are to be formed, the same conventional iterative sequence used in forming polynucleotides from nucleoside reagents on a support by means of known chemistry. This iterative sequence is as follows: (a) coupling a selected nucleoside through a phosphite linkage to a functionalized support in the first iteration, or a nucleoside bound to the substrate (i.e. the nucleoside-modified substrate) in subsequent iterations; (b) optionally, but preferably, blocking unreacted hydroxyl groups on the substrate bound nucleoside; (c) oxidizing the phosphite linkage of step (a) to form a phosphate linkage; and (d) removing the protecting group ("deprotection") from the now substrate bound nucleoside coupled in step (a), to generate a reactive site for the next cycle of these steps. The functionalized support (in the first cycle) or deprotected coupled nucleoside (in subsequent cycles) provides a substrate bound moiety with a linking group for forming the phosphite linkage with a next nucleoside to be coupled in step (a). Final deprotection of nucleoside bases can be accomplished using alkaline conditions such as ammonium hydroxide, in a known manner.

The foregoing chemistry of the synthesis of polynucleotides is described in detail, for example, in Caruthers, Science 230: 281-285, 1985; Itakura et al., Ann. Rev. Biochem. 53: 323-356; Hunkapillar et al., Nature 310: 105-110, 1984; and in "Synthesis of Oligonucleotide Derivatives in Design and Targeted Reaction of Oligonucleotide Derivatives", CRC Press, Boca Raton, Fla., pages 100 et seq., US 4,458,066, US 4,500,707, US 5,153,319, US 5,869,643, EP 0294196, and elsewhere. Suitable linking layers on the substrate include those as described in US 6,235,488 and 6,258,454 and the references cited therein.

Further details of fabricating biopolymer arrays by depositing either previously obtained biopolymers or by the *in situ* method are disclosed in US 6,242,266, US 6,232,072, US 6,180,351, and US 6,171,797.

In array fabrication, the quantities of polynucleotide or other biopolymer available, whether by deposition of previously obtained biopolymer or by *in situ* synthesis, are usually very small and expensive. Additionally, sample quantities available for testing are usually also very small and it is therefore desirable to simultaneously test the same sample against a large number of different probes on an array. These conditions require use of arrays with large numbers of very small, closely spaced features. When such arrays are read (such as by scanning them line by line with an illuminating light beam and recording any resulting fluorescence), large amounts of array signal data result which essentially provide a resulting signal value for each read region (such as a pixel) of the array. To make sense of this data, feature signal characteristics are then extracted from the array signal data. That is, read regions are identified as belonging to a particular feature. The extraction may also include one or more further steps, such as determining a background signal which must be subtracted from the read signal from a feature, determining outlier pixels or outlier features which should be excluded from an evaluation of results, and the like.

In a conventional configuration, an operator initiates line by line reading of an array by a scanner, and the array signal data is collected in a memory. The operator may then direct a same processor which controls the scanner to initiate feature extraction, and is prompted to help the processor locate corners, features, and/or other array characteristics, on a displayed image of the array signal data from scanning. Such operator input is conventionally needed since array features on the image are often poorly defined such as when a feature only weakly binds to a component in a sample to which the array has been exposed. The array signal data is then feature extracted by the same computer which controls the scanner, using the guidance input by the operator. When feature extraction is completed on an array, a next array is scanned and the process repeated for each array to be read in turn. Given that an array may contain thousands of features and each feature may result in ten, twenty or more pixels of array signal data, this operation of reading an array and completing feature extraction can be time consuming and require a high degree of operator input, in view of the large amounts of data which must be collected and processed. As a result, high throughput reading and feature extraction of arrays becomes difficult and time consuming in the conventional configuration. While one can purchase additional expensive scanners and their controlling computers, the conventional configuration still results in inefficient use of resources since the scanner or controlling computer may be waiting for the other to complete its operation (scanning or feature extraction), and operator input is used during feature extraction for each array.

It is desirable then to provide a means which makes good use of available resources to scan and feature extract multiple chemical arrays, to facilitate high throughput of the combined reading and feature extraction operations.

The present invention then, provides in one aspect a method which includes reading multiple chemical arrays (such as polynucleotide or peptide arrays) each having a plurality of features, to obtain array signal data. This data may then be saved in a memory. The saved signal data may be retrieved from the memory, and feature characteristics extracted therefrom. The saved signal data for an array may be extracted while another array is being read.

The chemical array saved signal data may be automatically retrieved from the memory at each of one or more processors as the processor becomes available to perform feature characteristic extraction on the retrieved signal data for the chemical array. For example, the feature extracting processor may signal the memory that it is available either upon its own initiative or in response to an inquiry. Each processor then automatically extracts feature characteristics from the retrieved signal data. This retrieval and extraction process may be automatically repeated by each of the one or more processors until all saved signal data for multiple chemical arrays in the memory has had feature characteristics extracted therefrom.

Multiple arrays may be read at each of one or more reading stations and the resulting array signal data saved in a common memory with which the reading stations communicate. Alternatively, or additionally, saved array signal data may be retrieved from a common memory at each of one or more processors which communicate with the common memory and each of which extracts feature characteristics from the retrieved array signal data.

Each of the read arrays may be associated with a corresponding identifier (for example, the identifier being present on the array substrate, a housing carrying the array, or in a same package carrying the array). In this case, the method may additionally include reading the array identifiers (such as at each of the reading stations) and saving each read array identifier in the memory in association with the saved array signal data for the corresponding array. For each array, the identifier may be retrieved from the memory in association with the retrieved array signal data, and extracted feature characteristics for the array saved in a memory in association with the retrieved identifier. This allows for later retrieving from the memory, the extracted feature characteristics for each of multiple arrays, based on the corresponding identifier for that array. For example, the method may additionally include, at a sample processing station, exposing an array to a sample and reading the associated array identifier. The array reading may then be performed at an array reading station, and extracted feature characteristics for each array retrieved based on the associated array identifier as read at the sample processing station.

In the case where multiple array reading stations communicate with the common memory, the method may additionally include for each of multiple arrays, saving a reading station identification or characteristic in the memory in association with the saved signal data for that array. This may occur at a hub station such as described below.

The present invention further provides for a method which may operate at a hub station, which method includes receiving the array signal data from multiple reading stations, saving the received array signal data in a memory, and retrieving saved array signal data from the memory and communicating the retrieved array signal data to multiple processors. The method executed at the hub may also include receiving an array identifier with the array signal data for each corresponding array and saving both in association with one another, as well as retrieving the array signal data based on a received communication of the identifier for the corresponding array. The hub may further receive from each of multiple reading stations, a reading station identification or characteristic (or both) in association with an array signal data, and save the reading station identification or characteristic in a memory in association with the saved signal data for that array.

The present invention further provides an apparatus which can execute any one or more methods of the present invention. In one aspect the apparatus includes a memory, an array reader having a first processor, and a second processor. The first processor communicates with the memory, and causes the reader to read multiple chemical arrays to obtain array signal data, and saves the read array signal data in the memory. The second processor communicates with the memory and retrieves saved signal data for arrays from the memory and extracts feature characteristics therefrom. Multiple first or second processor (or both) may be provided, each of which operates as just described and which communicates with the common memory. For example, in methods or apparatus of the present invention each first processor may be disposed at an array reader station and each second processor may be disposed at a processing station. Signal data for an array may be extracted while another array is being read by an array reader. The array reader may also include an identifier reader which for each array reads a corresponding array identifier associated with that array. In this case the first processor saves each read array identifier in the memory in association with the saved array signal data for the corresponding array. In another aspect, the apparatus includes a hub station which receives array signal data from multiple reading stations and saves that data in a memory, and also retrieves saved array signal data from the memory and communicates the retrieved array signal data to multiple processing stations upon receipt of an indication from each processing station that it is ready to process further array signal data.

The present invention further provides a computer program product for use with an apparatus of the present invention (for example, a user station, hub station, or any processing station). The program product includes a computer readable storage medium having a computer program stored thereon and which, when loaded into a programmable processor, provides instructions to the processor of that apparatus such that it will execute the procedures required of it to perform a method of the present invention.

The various aspects of the present invention can provide any one or more of the following and/or other useful benefits. For example, good use is made of available array reading and processing resources, so as to facilitate high throughput of the combined reading and feature extraction operations.

Embodiments of the invention will now be described with reference to the drawings, in which:
FIG. 1 illustrates a substrate carrying multiple arrays, such as may be fabricated by methods of the present invention;
FIG. 2 is an enlarged view of a portion of FIG. 1 showing ideal spots or features;
FIG. 3 is an enlarged illustration of a portion of the substrate in FIG. 2;
FIG. 4 illustrates a step in array feature extraction;
FIG. 5 shows an apparatus of the present invention; and
FIG. 6 is a flowchart illustrating a method of the present invention such as may be executed by the apparatus of FIG. 5.

To facilitate understanding, the same reference numerals have been used, where practical, to designate elements that are common to the Figures.

In the present application, unless a contrary intention appears, the following terms refer to the indicated characteristics. A "biopolymer" is a polymer of one or more types of repeating units. Biopolymers are typically found in biological systems and particularly include polysaccharides (such as carbohydrates), and peptides (which term is used to include polypeptides, and proteins whether or not attached to a polysaccharide) and polynucleotides as well as their analogs such as those compounds composed of or containing amino acid analogs or non-amino acid groups, or nucleotide analogs or non-nucleotide groups. This includes polynucleotides in which the conventional backbone has been replaced with a non-naturally occurring or synthetic backbone, and nucleic acids (or synthetic or naturally occurring analogs) in which one or more of the conventional bases has been replaced with a group (natural or synthetic) capable of participating in Watson-Crick type hydrogen bonding interactions. Polynucleotides include single or multiple stranded configurations, where one or more of the strands may or may not be completely aligned with another. A "nucleotide" refers to a sub-unit of a nucleic acid and has a phosphate group, a 5 carbon sugar and a nitrogen containing base, as well as functional analogs (whether synthetic or naturally occurring) of such sub-units which in the polymer form (as a polynucleotide) can hybridize with naturally occurring polynucleotides in a sequence specific manner analogous to that of two naturally occurring polynucleotides.. For example, a "biopolymer" includes DNA (including cDNA), RNA, oligonucleotides, and PNA and other polynucleotides as described in US 5,948,902 and references cited therein (all of which are incorporated herein by reference), regardless of the source. An "oligonucleotide" generally refers to a nucleotide multimer of about 10 to 100 nucleotides in length, while a "polynucleotide" includes a nucleotide multimer having any number of nucleotides. A "biomonomer" references a single unit, which can be linked with the same or other biomonomers to form a biopolymer (for example, a single amino acid or nucleotide with two linking groups one or both of which may have removable protecting groups). A "peptide" is used to refer to an amino acid multimer of any length (for example, more than 10, 10 to 100, or more amino acid units). A biomonomer fluid or biopolymer fluid reference a liquid containing either a biomonomer or biopolymer, respectively (typically in solution).

A "pulse jet" is a device which can dispense drops in the formation of an array. Pulse jets operate by delivering a pulse of pressure (such as by a piezoelectric or thermoelectric element) to liquid adjacent an outlet or orifice such that a drop will be dispensed therefrom. A "drop" in reference to the dispensed liquid does not imply any particular shape, for example a "drop" dispensed by a pulse jet only refers to the volume dispensed on a single activation. A drop which has contacted a substrate is often referred to as a "deposited drop" or the like, although sometimes it will be simply referenced as a drop when it is understood that it was previously deposited. Detecting a drop "at" a location, includes the drop being detected while it is traveling between a dispenser and that location, or after it has contacted that location (and hence may no longer retain its original shape) such as capturing an image of a drop on the substrate after it has assumed an approximately circular shape of a deposited drop.

A "set" or "sub-set" of any item (such as a set of arrays) may contain only one of the item, or only two, or three, or any multiple number of the items. An "array", unless a contrary intention appears, includes any one, two or three dimensional arrangement of addressable regions bearing a particular chemical moiety to moieties (for example, biopolymers such as polynucleotide sequences) associated with that region. An array is "addressable" in that it has multiple regions of different moieties (for example, different polynucleotide sequences) such that a region (a "feature" or "spot" of the array) at a particular predetermined location (an "address") on the array will detect a particular target or class of targets (although a feature may incidentally detect non-targets of that feature). Array features are typically, but need not be, separated by intervening spaces. In the case of an array, the "target" will be referenced as a moiety in a mobile phase (typically fluid), to be detected by probes ("target probes") which are bound to the substrate at the various regions. However, either of the "target" or "target probes" may be the one which is to be evaluated by the other (thus, either one could be an unknown mixture of polynucleotides to be evaluated by binding with the other). An "array layout" refers collectively to one or more characteristics of the features, such as feature positioning, one or more feature dimensions, and the chemical moiety or mixture of moieties at a given feature. "Hybridizing" and "binding", with respect to polynucleotides, are used interchangeably.

A "processor" references any hardware and/or software combination which will perform the functions required of it. For example, any processor herein may be a programmable digital microprocessor as available in the form of a personal desktop computer. Where the processor is programmable, suitable programming can be communicated from a remote location to the processor, or previously saved in a computer program product (such as a portable or fixed computer readable storage medium, whether magnetic, optical or solid state device based). For example, a magnetic or optical disk may carry the programming, and can be read by a suitable disk reader communicating with each processor at its corresponding station. When one item is indicated as being "remote" from another, this is referenced that the two items are at least in different rooms in a same building, in different buildings, and may be at least one mile, ten miles, or at least one hundred miles apart. Items that are not remote may at least be in a same room of a building, and may be within one hundred feet or even twenty feet, of one another. "Communicating" or "retrieving" information, or similar terms, references transmitting or retrieving the data representing that information as electrical signals over a suitable communication channel (for example, a private or public network). "Forwarding" an item refers to any means of getting that item from one location to the next, whether by physically transporting that item or otherwise (where that is possible) and includes, at least in the case of data, physically transporting a medium carrying the data or communicating the data.

It will also be appreciated that throughout the present application, that words such as "top", "upper", and "lower" are used in a relative sense only. "Fluid" is used herein to reference a liquid. Reference to a singular item, includes the possibility that there are plural of the same items present. "May" means optionally. Methods recited herein may be carried out in any order of the recited events which is logically possible, as well as the recited order of events. All patents and other cited references herein, are specifically incorporated into this application by reference except insofar as any may conflict with the present application (in which case the present application prevails).

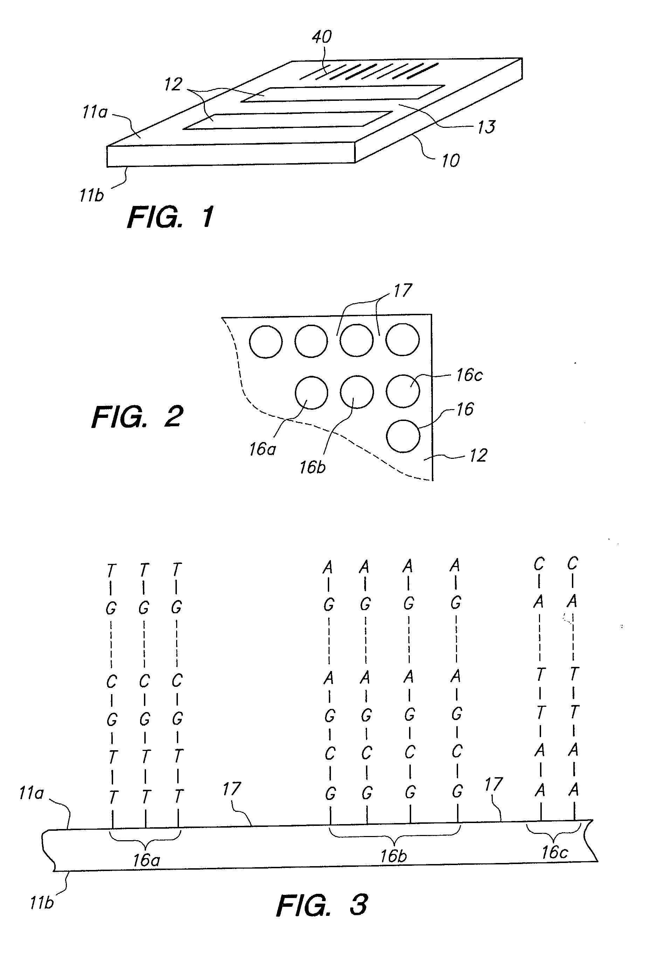

Referring first to FIGS. 1-3, typically methods and apparatus of the present invention generate or use a contiguous planar substrate 10 carrying one or more arrays 12 disposed across a front surface 11a of substrate 10 and separated by inter-array areas 13. A back side 11b of substrate 10 does not carry any arrays 12. The arrays on substrate 10 can be designed for testing against any type of sample, whether a trial sample, reference sample, a combination of them, or a known mixture of polynucleotides (in which latter case the arrays may be composed of features carrying unknown sequences to be evaluated). While two arrays 12 are shown in FIG. 1, it will be understood that substrate 10 may have any number of desired arrays 12. Arrays on any same substrate 10 may all have the same array layout, or some or all may have different array layouts. Similarly, substrate 10 may be of any shape, and any apparatus used with it adapted accordingly. Depending upon intended use, any or all of arrays 12 may be the same or different from one another and each will contain multiple spots or features 16 of biopolymers in the form of polynucleotides. A typical array may contain from more than ten, more than one hundred, more than one thousand or more than ten thousand features. All of the features 16 may be different, or some could be the same (for example, when any repeats of each feature composition are excluded the remaining features may account for at least 5%, 10%, or 20% of the total number of features). As best seen in FIG. 2, features 16 are arranged in straight line rows extending left to right in FIG. 2. In the case where arrays 12 are formed by the conventional *in situ* or deposition of previously obtained moieties, as described above, by depositing for each feature a droplet of reagent in each cycle such as by using a pulse jet such as an inkjet type head, interfeature areas 17 will typically be present which do not carry any polynucleotide or moieties of the array features. It will be appreciated though, that the interfeature areas 17 could be of various sizes and configurations. It will also be appreciated that there need not be any space separating arrays 12 from one another although there typically will be. Each feature carries a predetermined polynucleotide (which includes the possibility of mixtures of polynucleotides). As per usual, A, C, G, T represent the usual nucleotides. It will be understood that there may be a linker molecule (not shown) of any known types between the front surface 11a and the first nucleotide.

An array identifier 40 in the form of a bar code for both arrays 12 in FIG. 1, is associated with those arrays 12 to which it corresponds, by being provided on the same substrate 10 adjacent one of the arrays 12. A separate identifier can be provided adjacent each corresponding array 12 if desired. Identifier 40 may either contain information on the layout of array 12 or be linkable to a file containing such information in a manner such as described in US 6,180,351. Each identifier 40 for different arrays may be unique so that a given identifier will likely only correspond to one array 12 or to arrays 12 on the same substrate 10. This can be accomplished by making identifier 40 sufficiently long and incrementing or otherwise varying it for different arrays 12 or arrays 12 on the same substrate 10, or even by selecting it to be globally unique in a manner in which globally unique identifiers are selected as described in US 6,180,351.

Features 16 can have widths (that is, diameter, for a round feature 16) in the range of at least 10 µm, to no more than 1.0 cm. In embodiments where very small spot sizes or feature sizes are desired, material can be deposited according to the invention in small spots whose width is at least 1.0 µm, to no more than 1.0 mm, usually at least 5.0 µm to no more than 500 µm, and more usually at least 10 µm to no more than 200 µm. The size of features 16 can be adjusted as desired, during array fabrication. Features which are not round may have areas equivalent to the area ranges of round features 16 resulting from the foregoing diameter ranges.

For the purposes of the above description of FIGS. 1-3 and the discussions below, it will be assumed (unless the contrary is indicated) that the array being formed in any case is a polynucleotide array formed by the deposition of previously obtained polynucleotides using pulse jet deposition units. However, it will be understood that the described methods are applicable to arrays of other polymers (such as biopolymers) or chemical moieties generally, whether formed by multiple cycle *in situ* methods using precursor units for the moieties desired at the features, or deposition of previously obtained moieties, or using other types of dispensers. Thus, in those discussions "polynucleotide", "polymer" (such as "biopolymer") or "chemical moiety", can generally be interchanged with one another (although where specific chemistry is referenced the corresponding chemistry of an interchanged moiety should be referenced instead). It will also be understood that when methods such as an *in situ* fabrication method are used, additional steps may be required (such as oxidation and deprotection in which the substrate 10 is completely covered with a continuous volume of reagent).

Arrays such as those of FIGS. 1-3 can be fabricated using drop deposition from pulse jets of either polynucleotide precursor units (such as monomers) in the case of *in situ* fabrication, or the previously obtained polynucleotide. Such methods are described in detail in, for example, the previously cited references including US 6,242,266, US 6,232,072, US 6,180,351, US 6,171,797, US 6,323,043, U.S. Patent Application Serial No. 09/302,898 filed April 30, 1999 by Caren et al., and the references cited therein. As already mentioned, these references are incorporated herein by reference. Other drop deposition methods can be used for fabrication, as previously described herein. Also, instead of drop deposition methods, other array fabrication method may be used such as described in US 5,599,695, US 5,753,788, and US 6,329,143.

Following receipt by a user receives of an array 12, it will typically be exposed to a sample (for example, a fluorescently labeled polynucleotide or protein containing sample) and the array then read to obtain the resulting array signal data.
Reading of the array may be accomplished by illuminating the array and reading the location and intensity of resulting fluorescence at each feature of the array,. For example, a scanner may be used for this purpose which is similar to the AGILENT MICROARRAY SCANNER manufactured by Agilent Technologies, Palo Alto, CA. Other suitable apparatus and methods are described in U.S. patent applications: Serial No. 09/846125 "Reading Multi-Featured Arrays" by Dorsel et al.; and Serial No. 09/430214 "Interrogating Multi-Featured Arrays" by Dorsel et al. However, arrays may be read by any other method or apparatus than the foregoing, with other reading methods including other optical techniques (for example, detecting chemiluminescent or electroluminescent labels) or electrical techniques (such as where each feature is provided with an electrode to detect hybridization at that feature in a manner disclosed in US 6,251,685, US 6,221,583 and elsewhere). Results from the reading may be raw results (such as fluorescence intensity readings for each feature in one or more color channels) or may be processed results such as obtained by rejecting a reading for a feature which is below a predetermined threshold and/or forming conclusions based on the pattern read from the array (such as whether or not a particular target sequence may have been present in the sample, or whether or not a pattern indicates a particular condition of an organism from which the sample came). The results of the reading (processed or not) may be forwarded (such as by communication) to a remote location if desired, and received there for further use (such as further processing).

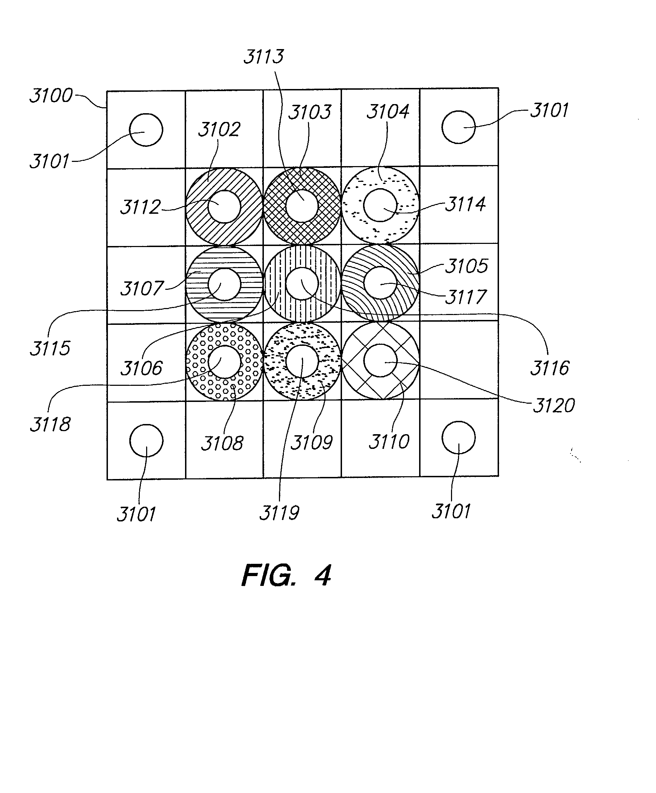

In order to make sense of the read array signal data one or more feature signal characteristics are then evaluated in a feature extraction operation. In typical feature extraction pixels in the array signal data are identified as belonging to particular array features. One way of accomplishing this illustrated in FIG. 4. For simplicity, FIG. 4 illustrates feature extraction on an array of nine features. However, the same principle can be applied to any size array. In particular, for arrays with features arranged in rows and columns, corners 3101 or other features of the array in the array signal image can be located using any one or more of: fiducials (not shown) provided on substrate 10, such as in a manner described in US 5,721,435; the features in the signal image at the array corners themselves; or a method such as described in detail in U.S. patent application Serial No. 09/659,415 titled "Method And System For Extracting Data From Surface Array Deposited Features" filed by Enderwick et al. on Sept. 11, 2000 (and also in European Patent Application publication EP 1162572). Based on location of the comers a rectilinear grid 3100 can then be established in the array signal image (and optionally refined using the center of regions of strongest signals), and the expected locations 3112 to 3120 of features in the signal data image determined. The present invention is able to make use of the fact that with such techniques for finding features on an array signal image, little or no operator input is needed to find array features or other locations on the array signal image, such that feature extraction can be automated. This ability to automate feature extraction with little or no operator input to aid in the extraction process, allows the feature extraction process to be rapid and enhances the use of the present invention.

Note that the expected size of each feature can be retrieved as part of the array layout using array identifier 40 in a manner as described in US 6,180,351 whether identifier 40 is a local identifier or is itself a globally unique identifier described therein. As an additional part of the feature extraction operation, regions 3108 through 3110 in the signal image around each determined feature location 3112-3120 between those feature locations and grid 3100 can be determined as background regions, the signal from those regions evaluated to provide an average pixel background signal, and this background signal subtracted from each pixel signal within the determined features locations 3112-3120. The foregoing feature extraction procedure is described in detail in U.S. Patent Application Serial No. 09/659,415 previously referenced. As a further part of the feature extraction operation, the presence of outlier pixels and features can be evaluated in a manner described in U.S. Provisional Patent Application Serial No. 60/268,115, entitled "Algorithm For The Detection Of Intra-Feature Heterogeneity Outliers" filed Feb. 9, 2001 by Delenstarr. As already mentioned, these cited references are incorporated herein by reference.

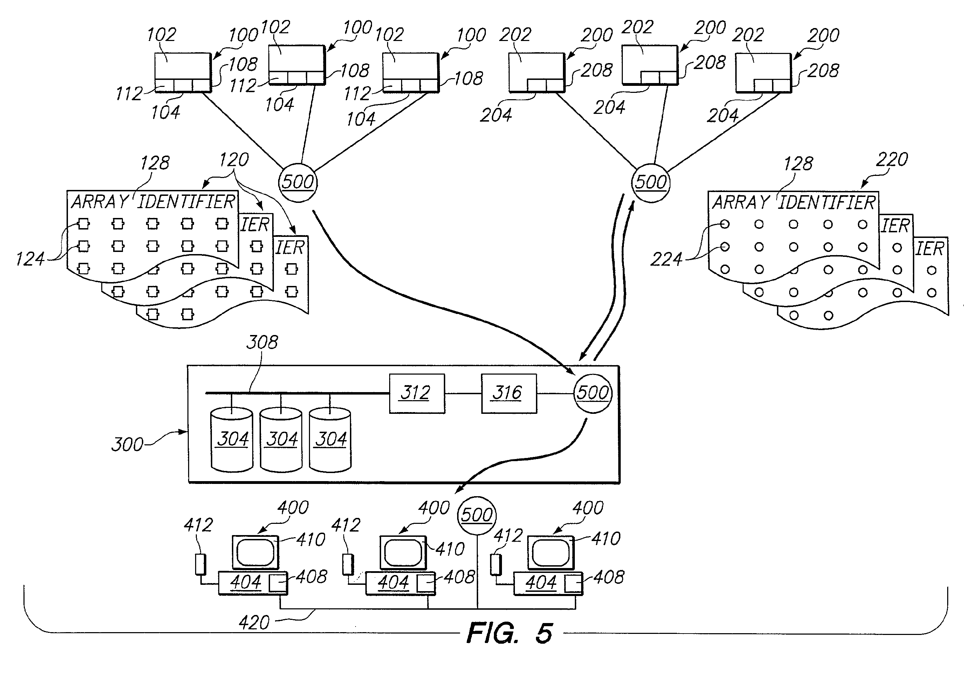

Turning now to FIG. 5, an apparatus of the present invention will be described.
The apparatus includes multiple array reader stations 100, each having an array reader 102 which includes a first processor 104 and a communication module 108 through which each first processor 104 can communicate over a communication network 500 with a central memory 300 in the form of a hub station. Each array reader station 100 further has an identifier reader 112, such as a bar code reader, capable of reading identifiers 40. Hub station 300 includes multiple memory devices 304 (such as hard disk drives or optical disk drives) which communicate over a common data bus with a processor 312 which can also communicate over network 500 through a communication module 316. Thus, hub station 300 appears to the other stations as one central memory although it may contain any number of memory devices 304. Multiple processor stations 200, each have a second processor 204 and a communication module 208 through which each second processor can also communicate over communication network 500 with hub station. Multiple user stations 400 each have a third processor 404 which also can communicate over communication network 500 through a communication module 408. Each user station 400 further has an identifier reader 412, such as a bar code reader, capable of reading identifiers 40. Each user station 400 may also serve as a sample processing station, as will shortly be described, although separate stations could be provided for user stations and sample processing stations.

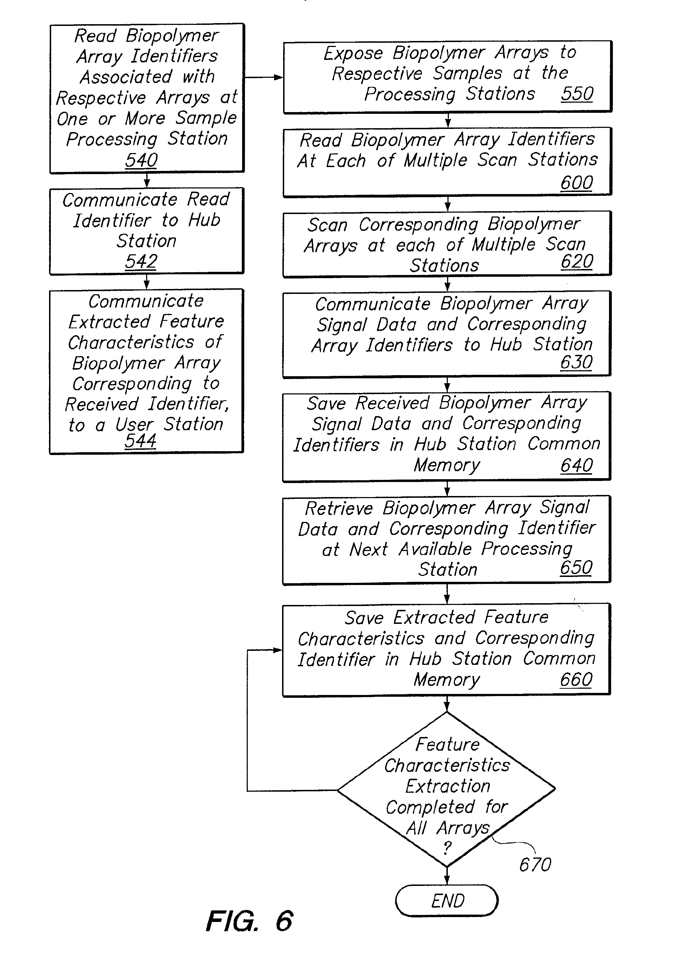

Referring to FIGS. 5 and 6, the operation of the apparatus of FIG. 5 in accordance with a method of the present invention will now be described. Reference numbers in parentheses refer to FIG. 6. It will be assumed that all processors are programmed as needed to execute the steps required of it at each station. First, at each user station 400 a user will cause the user station 400 to read (540) identifiers 40 associated with respective multiple arrays 12 by passing each identifier 40 beneath identifier reader 412. The read identifiers can then be saved in a local memory (not shown) at each user station 400. The user will then expose (550) multiple arrays 12 to respective samples at user station 400. However, it will be appreciated that the order of identifier reading (540) and sample exposure (550) can be reversed or can be simultaneous. Following sample exposure (550) and washing and optional drying of the exposed arrays 12, the exposed arrays 12 are forwarded from each user station 400 to any one or more array reader stations 100.

Multiple exposed arrays 12 received at each user station then have their associated identifiers 40 corresponding to each array 12, read (600) by identifier reader 112. Each of those received arrays 12 may then be read (620) by array reader 102 at each reader station 100. The reading at each reader station may be automatic with any needed parameters required for the reading (such as area to be scanned, light source intensity) being retrieved based on bar code 40 in a manner such as described in US 6,180,351, and U.S. Patent Application Serial No. 09/302,898 for "Polynucleotide Array Fabrication" filed April 30, 1999 and owned by the same assignee as the present application (and British Patent Publication GB 2355716). The resulting array signal data may then be communicated (630) along with corresponding identifiers (also now in electronic data form), to hub station 300 over network 500. The communicated data may be in the form of digital files 120 illustrated schematically in FIG. 5, each named with an array identifier 128 and carrying the array signal data 124 for the corresponding array 12 (that is, the array physically associated with that identifier, such as by being in proximity on the same substrate). Each such file 120 may then be saved (640) at hub station 300 such that the array signal data 124 for a given array can then be retrieved based on the file name in the form of identifier 128.

Each reader station 100 may also communicate, as part of each file 120, a unique identifier of that reader station 100 (such as "READER STATION XXX" where XXX is a unique alphanumeric identifier), or one or more characteristics of that reader station 100. Such characteristics may include any one or more of model and make of the reader, illuminating light intensity for one or more features, sensitivity characteristics of a sensor which detects the signal from the array (such as sensitivity or voltage characteristics of a fluorescence detector, such as a photomultiplier tube or charge coupled device sensor), or any other characteristic of the means by which the array was read. Since such identifier or characteristics are in the same file 120 as the corresponding array signal data, they are all associated with one another.

Each processing station 200 automatically retrieves array signal 124 data by signaling its availability to perform feature extraction on array signal data 124, to hub station 300. A file 120 is then retrieved (650) at the next available processing station 200, and feature extraction is automatically performed on the array signal data 124 without the need for operator input into the extraction operation. When extraction of data 124 is completed for a file 120, the extracted feature signal characteristic data 224 is added to file 120 to thereby form file 220 which is then communicated back to hub station 300 at which it is saved (660). Note that file 220 will bear the same name (identifier 128) and also continue to carry any further information originally present in file 120 (including any reader station identifier or characteristics, and the original array signal data 124). This process may be automatically repeated multiple times at the hub station and each processing station 100, with each processing station 100 signaling its availability to hub station 300 (either on its own initiative on in response to a query from hub station 300), until all arrays have been feature extracted (670).

Furthermore, any user station 400 may communicate (542) a read identifier for an exposed array, previously saved in local memory, to hub station 300. This will constitute a request to hub station 300 to communicate the feature extracted data 224 to the requesting user station 400, which corresponds to the identifier received from that user station 400. Hub station 300 can compare the identifier with the identifier 128 in any of the saved files 220 of feature extracted data. If a match is found, hub station 300 can retrieve the corresponding feature extracted data 224 from memory based on the received identifier from the user station 400 and communicate (544) that to the requesting user station 400. If a match is not found, hub station 300 can so inform the requesting user station so that a user can make the same request with hub station 300 at a later time (after which a processing station 100 may have feature extracted the corresponding array). Alternatively, hub station 300, after so informing the user, can automatically check its memory periodically to see if the corresponding feature extracted data 224 has been received and, when received, then communicate it to the requesting user station 400.

Additionally, if each scanner station 100 saves in a local memory (not shown) at that scanner station 100, a first list of identifiers for all arrays 12 which it has read and for which array signal data has been communicated to hub station 300, then a user station can communicate an array identifier to one or more scanner stations as a confirmation request as to whether one of those reader stations has yet read the corresponding array and communicated the array signal data to hub station 300. In this case each scanner station 100 receiving such a confirmation request need only check the received communicated array identifier against its locally saved first list, and respond in the affirmative/negative if that identifier is/is not on the locally saved first list. As a further option, each reader station 100 can read each array identifier 40 as the arrays 12 are received and before reading, and save such read identifiers in local memory in a second list. When a confirmation request is received from a user station 400 the received identifier can also be checked against the second locally saved list at that user station 400, and a response communicated to the requesting user station 400 that the corresponding array was/was not received at that reader station 100 if that identifier is/is not on the locally saved second list.

Note that during operation of the above method, the array reading at each reading station can be performed automatically based on array layout information retrieved using identifier 40 for a corresponding array, as described above. The saved array signal data for one or more arrays may be feature extracted at a processing station 200 while one or more other arrays are being read at a reading station 100. Further, this extraction may also be performed automatically based on array layout information retrieved using identifier 40 as described in US 6,180,351, and methods such as described in U.S. Patent Application Serial No. 09/302,898, both incorporated herein by reference. Thus, array reading and feature extraction can become automatic and independent operations without one waiting for the other, and without waiting for operator input to aid in the extraction operation. Additionally, the apparatus and method can be reduced or expanded, with additional array readers 100, processor stations 300, or user stations 400, being added or deleted to meet demand or changes in speed at one or more of the other stations. Furthermore, when a reader or processor identifier or characteristics are present in files 220 saved at hub station 300, a user at a user station 400 which retrieves such a file 220 can examine the file for potential problem characteristics which may shed light on suspect feature extraction data 224. Such problem characteristics may include, in the case of a reader 100, low detector sensitivity, older model reader, and the like, and in the case of a processor 200, an old version of a feature extraction algorithm, questionable extraction algorithm parameter settings, and the like.

It will also be appreciated that any of the array readers 100 may be remote or not from one another. This is also true for any of the processing stations 200 as well as any of the user stations 400. Furthermore, any group of array readers 100, processing stations 200, and user stations 400, and hub station 300, may or may not be remote from one another. As well, any of the networks described herein may be local, wide area networks, and may include communication over wire, wireless, or optical communication channels, or any combination of the foregoing.

The present methods and apparatus may be used with biopolymers or other chemical moieties on surfaces of any of a variety of different substrates, including both flexible and rigid substrates. Preferred materials provide physical support for the deposited material and endure the conditions of the deposition process and of any subsequent treatment or handling or processing that may be encountered in the use of the particular array. The array substrate may take any of a variety of configurations ranging from simple to complex. Thus, the substrate could have generally planar form, as for example a slide or plate configuration, such as a rectangular or square or disc. In many embodiments, the substrate will be shaped generally as a rectangular solid, having a length in the range about 4 mm to 1 m, usually about 4 mm to 600 mm, more usually about 4 mm to 400 mm; a width in the range about 4 mm to 1 m, usually about 4 mm to 500 mm and more usually about 4 mm to 400 mm; and a thickness in the range about 0.01 mm to 5.0 mm, usually from about 0.1 mm to 2 mm and more usually from about 0.2 to 1 mm. However, larger substrates can be used, particularly when such are cut after fabrication into smaller size substrates carrying a smaller total number of arrays 12.

In the present invention, any of a variety of geometries of arrays on a substrate 10 may be used. For example, arrays 12 can be arranged in a sequence of curvilinear rows across the substrate surface (for example, a sequence of concentric circles or semi-circles of spots), or in some other arrangement. Similarly, the pattern of features 16 may be varied from the rectilinear rows and columns of spots in FIG. 2 to include, for example, a sequence of curvilinear rows across the substrate surface (for example, a sequence of concentric circles or semi-circles of spots), or some other regular pattern. Even irregular arrangements are possible provided a user is provided with some means (for example, an accompanying description) of the location and an identifying characteristic of the features (either before or after exposure to a sample). The configuration of the arrays and their features may be selected according to manufacturing, handling, and use considerations.

The array substrates 10 may be fabricated from any of a variety of materials. In certain embodiments, such as for example where production of binding pair arrays for use in research and related applications is desired, the materials from which the substrate may be fabricated should ideally exhibit a low level of non-specific binding during hybridization events. In many situations, it will also be preferable to employ a material that is transparent to visible and/or UV light. For flexible substrates, materials of interest include: nylon, both modified and unmodified, nitrocellulose, polypropylene, and the like, where a nylon membrane, as well as derivatives thereof, may be particularly useful in this embodiment. For rigid substrates, specific materials of interest include: glass; plastics (for example, polytetrafluoroethylene, polypropylene, polystyrene, polycarbonate, and blends thereof, and the like); metals (for example, gold, platinum, and the like).

The substrate surface onto which the polynucleotide compositions or other moieties is deposited may be smooth or substantially planar, or have irregularities, such as depressions or elevations. The surface may be modified with one or more different layers of compounds that serve to modify the properties of the surface in a desirable manner. Such modification layers, when present, will generally range in thickness from a monomolecular thickness to about 1 mm, usually from a monomolecular thickness to about 0.1 mm and more usually from a monomolecular thickness to about 0.001 mm. Modification layers of interest include: inorganic and organic layers such as metals, metal oxides, polymers, small organic molecules and the like. Polymeric layers of interest include layers of: peptides, proteins, polynucleic acids or mimetics thereof (for example, peptide nucleic acids and the like); polysaccharides, phospholipids, polyurethanes, polyesters, polycarbonates, polyureas, polyamides, polyethyleneamines, polyarylene sulfides, polysiloxanes, polyimides, polyacetates, and the like, where the polymers may be hetero- or homopolymeric, and may or may not have separate functional moieties attached thereto (for example, conjugated).

Other embodiments of the present invention may include the following.

An apparatus comprising:
a) a common memory;
b) multiple array reading stations, each having a first processor which communicates with the common memory, wherein the first processor causes the reader to read multiple chemical arrays each having a plurality of features, to obtain array signal data, and saves the read array signal data in the common memory; and
c) multiple processing stations, each having a second processor which communicates with the memory and which retrieves saved signal data for arrays from the memory and extracts feature characteristics therefrom.

An apparatus according to the foregoing, wherein
each array reading station includes an identifier reader which for each array reads a corresponding array identifier associated with that array; and
the first processor of each array reader saves each read array identifier in the common memory in association with the saved array signal data for the corresponding array.

An apparatus according to the foregoing wherein for each array the second processor retrieves the identifier from the memory in association with the retrieved array signal data, and saves extracted feature characteristics for the array in a memory in association with the retrieved identifier.

An apparatus according to the foregoing wherein each identifier reader reads array identifiers carried on an array substrate or a housing carrying the array.

An apparatus according to the foregoing a hub which:
a) receives from multiple reading stations, array signal data from the reading of multiple chemical arrays each having a plurality of features, and saves the received array signal data from the multiple reading stations in a memory; and
c) retrieves saved array signal data for arrays from the memory and communicates the retrieved array signal data to multiple processors upon receipt of an indication from each processor that it is ready to process further array signal data.

An apparatus according to the foregoing wherein the array signal data for each array is retrieved by the hub based on a received communication of the identifier for the corresponding array.

An apparatus according to the foregoing wherein, for each of multiple reading stations, the hub receives a reading station identification or characteristic in association with an array signal data, and saves the received reading station identification or characteristic in a memory in association with the saved signal data for that array.

A method comprising forwarding data representing a result of a reading and extracting obtained by a method .

A method according to the foregoing wherein the data is communicated to a remote location.

A method comprising receiving data representing a result of a reading and extracting obtained by a method herein.

In one aspect of the invention there is provided a method comprising:
a) reading at each of multiple reading stations, multiple chemical arrays each having a plurality of features, to obtain array signal data;
b) saving the array signal data from the multiple reading stations in a common memory;
c) automatically retrieving saved signal data for chemical arrays from the common memory at each of one or more processors communicating with the common memory, as each processor becomes available to perform feature characteristic extraction on the retrieved signal data for the chemical array, and extracting feature characteristics from the retrieved chemical array signal data at each of the processors.

Preferably, there are multiple processing stations communicating with the common memory, each of which retrieves chemical array saved signal data from the common memory.

Preferably, each of the read arrays is associated with a corresponding identifier, the method additionally comprising reading the array identifiers at each of the multiple reading stations and saving each read array identifier in the common memory in association with the saved array signal data for the corresponding array.

The associated array identifiers may be on the array substrate, a housing carrying the array, or in a same package carrying the array.

The method may additionally comprise for each of multiple arrays:
retrieving the identifier from the common memory in association with the retrieved array signal data, and saving extracted feature characteristics for the array in a memory in association with the retrieved identifier.

In another aspect there is provided a method comprising:
a) reading at each of one or more reading stations, multiple chemical arrays each having a plurality of features, to obtain array signal data;
b) saving the array signal data from the one or more reading stations in a common memory;
c) retrieving saved signal data for chemical arrays from the common memory at each of multiple processing stations communicating with the common memory, and extracting feature characteristics from the retrieved chemical array signal data at each of the processing stations.

In another aspect there is provided a method comprising:
a) receiving at a hub station from multiple reading stations, array signal data from the reading of multiple chemical arrays each having a plurality of features;
b) saving the received array signal data from the multiple reading stations in a memory;
c) retrieving saved array signal data for arrays from the memory and communicating the retrieved array signal data to multiple processing stations.

The method may comprise receiving an array identifier with the array signal data for each corresponding array and saving both in association with one another.

The array signal data for each array may be retrieved based on a received communication of the identifier for the corresponding array.

The method preferably additionally comprises, for each of multiple reading stations, receiving a reading station identification or characteristic at the hub station in association with an array signal data, and saving the received reading station identification or characteristic in a memory in association with the saved signal data for that array.

Various further modifications to the particular embodiments described above are, of course, possible. Accordingly, the present invention is not limited to the particular embodiments described in detail above.

## Claims

1. A method comprising:
a) reading multiple chemical arrays 12 each having a plurality of features 16, to obtain array signal data;
b) saving the array signal data for the multiple arrays in a memory 300;
c) retrieving saved signal data for chemical arrays from the memory and extracting feature characteristics therefrom, wherein the saved signal data for a chemical array is extracted while another chemical array is being read.

2. A method as claimed in claim 1 wherein the arrays are polynucleotide or peptide arrays.

3. A method as claimed in claim 1 or 2 wherein the chemical array saved signal data is automatically retrieved from the memory at each of one or more processors as the processor becomes available to perform feature characteristic extraction on the retrieved signal data for the chemical array, and extracts feature characteristics from the retrieved signal data.

4. A method as claimed in claim 3 wherein the retrieval and extraction of saved signal data for a chemical array are automatically repeated by each of the one or more processors until all saved signal data for multiple chemical arrays in the memory has had feature characteristics extracted therefrom.

5. A method as claimed in any preceding claim wherein each of the read arrays is associated with a corresponding identifier 40, the method additionally comprising reading the array identifiers and saving each read array identifier in the memory in association with the saved array signal data for the corresponding array.

6. A method as claimed in claim 5 additionally comprising for each array:
retrieving the identifier from the memory in association with the retrieved array signal data, and saving extracted feature characteristics for the array in a memory in association with the retrieved identifier.

7. A method as claimed in claim 6 additionally comprising retrieving extracted feature characteristics for each of multiple arrays based on the corresponding identifier for that array.

8. A method as claimed in claim 5 to 7 wherein the associated array identifiers are on the array substrate 10, a housing carrying the array, or in a same package carrying the array.

9. A method as claimed in claim 5 to 8 additionally comprising
at a sample processing station, exposing an array to a sample and reading the associated array identifier;
wherein the array reading is performed at an array reading station and extracted feature characteristics for each array are retrieved based on the associated array identifier as read at the sample processing station.

10. A method as claimed in any preceding claim wherein the arrays are read at multiple reading stations, the method additionally comprising for each of multiple arrays, saving a reading station identification or characteristic in the memory in association with the saved signal data for that array.

11. A method as claimed in any preceding claim additionally comprising saving a processor identification or feature extraction characteristic in a memory in association with the extracted feature characteristics for each of the chemical arrays.

12. An apparatus comprising:
a) a memory 300;
b) an array reader 102 having a first processor 104 which communicates with the memory, wherein the first processor causes the reader to read multiple chemical arrays 12 each having a plurality of features 16, to obtain array signal data, and saves the read array signal data in the memory; and
c) a second processor 204 communicating with the memory and which retrieves saved signal data for arrays from the memory and extracts feature characteristics therefrom,
wherein the saved signal data for an array is extracted while another array is being read by an array reader.

13. An apparatus as claimed in claim 12 wherein the second processor automatically retrieves saved signal data for a chemical array from the memory as the processor becomes available to perform feature characteristic extraction on the retrieved signal data for the chemical array, and extracts feature characteristics from the retrieved signal data.

14. An apparatus as claimed in claim 12 or 13 wherein:
the array reader includes an identifier reader 112 which for each array reads a corresponding array identifier 40 associated with that array; and
the first processor saves each read array identifier in the memory in association with the saved array signal data for the corresponding array.

15. An apparatus as claimed in claim 14 wherein for each array the second processor retrieves the identifier from the memory in association with the retrieved array signal data, and saves extracted feature characteristics for the array in a memory in association with the retrieved identifier.

16. An apparatus as claimed in claim 15 additionally comprising a user station 400 including a third processor 404 which communicates with the memory in which extracted feature characteristics and associated identifiers are saved and retrieves therefrom extracted feature characteristics for each of multiple arrays based on the corresponding identifier for that array.

17. An apparatus as claimed in any of claims 14 to 16 wherein the identifier reader reads associated array identifiers from an array substrate or a housing carrying the array.

18. An apparatus as claimed in any of claims 12 to 17 wherein:
the apparatus has multiple array readers each having a corresponding first processor which communicates with the same common memory, wherein each first processor causes the corresponding reader to read multiple chemical arrays each having a plurality of features, to obtain array signal data, and saves the read array signal data in the common memory; and
each first processor of each array reader saves a reading station identification or characteristic in the common memory in association with the saved signal data for each array read at corresponding array reader.
